# EUROPEAN PATENT APPLICATION

(11) **EP 2 774 990 A1**
(43) Date of publication of application: **10.09.2014**
(21) Application number: 13158379.1
(22) Date of filing: 08.03.2013
(51) Int. Cl.: C12N 15/113, A61K 31/713

(54) **Loop-shRNAs**

(71) Applicant: Dr. Diederichs Lifre Science GmbH, 69121 Heidelberg (DE)
(72) Inventor: Diederichs, Sven, 69121 Heidelberg (DE)
(74) Representative: Dick, Alexander

(57) **Abstract**

The present invention relates to a polynucleotide forming a stem-loop structure under physiological conditions, wherein the loop comprises at least 80% of a sequence complementary or essentially complementary to a sequence of at least 15 contiguous nucleotides of an RNA expressed from a gene of interest; to a vector comprising or encoding said polynucleotide; and to a host cell comprising said polynucleotide and/or said vector. The present invention further relates to a use of a polynucleotide, a vector, or a host cell according to the present invention for treating disease; and to a method of inhibiting an unwanted cell population, comprising contacting said cell population with a polynucleotide, a vector, or a host cell according to the present invention, and thereby inhibiting an unwanted cell population. Further, the present invention relates to a kit comprising a polynucleotide, a vector, or a host cell according to the present invention and an instruction manual; and to a device comprising a polynucleotide, a vector, or a host cell according to the present invention and, preferably, means for applying said polynucleotide, vector, and/or host cell to the body of a subject.

SEQUENCE LISTING

<110>  Dr. Diederichs Life Science GmbH

<120>  Loop-shRNAs

<130>  DIE11777EP

<160>  16

<170>  PatentIn version 3.5

<210>  1
<211>  62
<212>  RNA
<213>  Homo sapiens

<400>  1
gugcauugua guugcauugc auguucuggu gguacccaug caauguuucc acagugcauc      60

ac                                                                    62

<210>  2
<211>  19
<212>  RNA
<213>  Homo sapiens

<400>  2
uguucuggug guacccaug                                                  19

<210>  3
<211>  64
<212>  RNA
<213>  Homo sapiens

<400>  3
uggcaguguc uuagcugguu guugugagca auaguaagga agcaaucagc aaguauacug      60

cccu                                                                  64

<210>  4
<211>  20
<212>  RNA
<213>  Homo sapiens

<400>  4
ugugagcaau aguaaggaag                                                 20

<210>  5
<211>  65
<212>  RNA
<213>  Homo sapiens

<400>  5
cugaccuaug aauugacagc cagugcucuc gucuccccuc uggcugccaa uuccauaggu      60

cacag                                                                 65

<210>  6
<211>  22
<212>  RNA
<213>  Homo sapiens

<400>  6
agugcucucg ucuccccucu gg                                                22


<210>  7
<211>  65
<212>  RNA
<213>  Homo sapiens

<400>  7
ugauugucca aacgcaauuc uuguacgagu cugcggccaa ccgagaauug uggcuggaca        60

ucugu                                                                   65


<210>  8
<211>  22
<212>  RNA
<213>  Homo sapiens

<400>  8
uguacgaguc ugcggccaac cg                                                22


<210>  9
<211>  64
<212>  DNA
<213>  Artificial Sequence

<220>
<223>  DNA encoding artificial shRNA partially complementary to
       Luciferase RNA

<400>  9
tgattgtcca aacgcaattc tgatttgtat tcagcccata tcagaattgt ggctggacat        60

ctgt                                                                    64


<210>  10
<211>  21
<212>  RNA
<213>  Artificial Sequence

<220>
<223>  loop-RNA, complementary to Luciferase RNA

<400>  10
gauuuguauu cagcccauau c                                                 21


<210>  11
<211>  64
<212>  DNA
<213>  Artificial Sequence

<220>
<223>  DNA encoding artificial shRNA partially complementary to

Luciferase RNA

<400>  11
tgattgtcca aacgcaattc ttgatacctg gcagatggaa ccagaattgt ggctggacat      60

ctgt                                                                    64


<210>  12
<211>  21
<212>  RNA
<213>  Artificial Sequence

<220>
<223>  loop-RNA, complementary to Luciferase RNA

<400>  12
ugauaccugg cagauggaac c                                                 21


<210>  13
<211>  64
<212>  DNA
<213>  Artificial Sequence

<220>
<223>  DNA encoding artificial shRNA partially complementary to
       Luciferase RNA

<400>  13
tgattgtcca aacgcaattc tgctatgtct ccagaatgta gcagaattgt ggctggacat      60

ctgt                                                                    64


<210>  14
<211>  21
<212>  RNA
<213>  Artificial Sequence

<220>
<223>  loop-RNA, complementary to Luciferase RNA

<400>  14
gcuaugucuc cagaauguag c                                                 21


<210>  15
<211>  65
<212>  DNA
<213>  Artificial Sequence

<220>
<223>  DNA encoding artificial shRNA partially complementary to
       Luciferase RNA

<400>  15
tgattgtcca aacgcaattc tcttaatcag agacttcagg cggagaattg tggctggaca      60

tctgt                                                                   65


<210>  16

<211>  22
<212>  RNA
<213>  Artificial Sequence

<220>
<223>  loop-RNA, complementary to Luciferase RNA

<400>  16
cuuaaucaga gacuucaggc gg                                                22

## Description

The present invention relates to a polynucleotide forming a stem-loop structure under physiological conditions, wherein the loop comprises at least 80% of a sequence complementary or essentially complementary to a sequence of at least 15 contiguous nucleotides of an RNA expressed from a gene of interest; to a vector comprising or encoding said polynucleotide; and to a host cell comprising said polynucleotide and/or said vector. The present invention further relates to a use of a polynucleotide, a vector, or a host cell according to the present invention for treating disease; and to a method of inhibiting an unwanted cell population, comprising contacting said cell population with a polynucleotide, a vector, or a host cell according to the present invention, and thereby inhibiting an unwanted cell population. Further, the present invention relates to a kit comprising a polynucleotide, a vector, or a host cell according to the present invention and an instruction manual; and to a device comprising a polynucleotide, a vector, or a host cell according to the present invention and, preferably, means for applying said polynucleotide, vector, and/or host cell to the body of a subject.

Small RNAs are important post-transcriptional regulators of gene expression. Endogenously, microRNAs (miRNAs) are generated through processing of long pri-miRNA precursors by Drosha and Dicer into a miRNA duplex (reviewed in Winter, J., Jung, S., Keller, S., Gregory, R.I. and Diederichs, S. (2009) Many roads to maturity: microRNA biogenesis pathways and their regulation. Nat Cell Biol, 11, 228-234.). The miRNA duplex forms a complex with Argonaute (Ago) proteins. The duplex is unwound giving rise to the active single-stranded miRNA in the RNA Induced Silencing Complex (RISC) that targets mRNAs for degradation or translational inhibition. Exogenous short interfering RNA (siRNA) or short hairpin RNA (shRNA) can be delivered into the cell as a miRNA duplex or hairpin, respectively, to silence specific target genes. Despite the different biogenesis pathways of endogenous and ectopic small RNAs, they all share one common feature: In the end, they form a double-stranded short RNA duplex that gives rise mostly to one active guide strand which represents the mature small RNA. While exogenous single-stranded siRNAs have been shown to be able to elicit an RNA interference (RNAi) response "bypassing" the microRNA biogenesis pathway (Martinez, J., Patkaniowska, A., Urlaub, H., Luhrmann, R. and Tuschl, T. (2002) Singlestranded antisense siRNAs guide target RNA cleavage in RNAi. Cell, 110, 563-574, Schwarz, D.S., Hutvagner, G., Haley, B. and Zamore, P.D. (2002) Evidence that siRNAs function as guides, not primers, in the Drosophila and human RNAi pathways. Mol Cell, 10, 537-548), chemical modifications to stabilize and activate these ss-siRNAs in vivo have only recently been described (Yu, D., Pendergraff, H., Liu, J., Kordasiewicz, H.B., Cleveland, D.W., Swayze, E.E., Lima, W.F., Crooke, S.T., Prakash, T.P. and Corey, D.R. (2012) Single-Stranded RNAs Use RNAi to Potently and Allele-Selectively Inhibit Mutant Huntingtin Expression. Cell, 150, 895-908, Lima, W.F., Prakash, T.P., Murray, H.M., Kinberger, G.A., Li, W., Chappell, A.E., Li, C.S., Murray, S.F., Gaus, H., Seth, P.P. et al. (2012) Single-Stranded siRNAs Activate RNAi in Animals. Cell, 150, 883-894.).

MiRNAs have been found to have global regulatory functions, the scope of which still is poorly understood. However, in modern molecular therapy approaches, it is frequently desirable to have the possibility to modulate two or more genes involved in disease, however without interfering with global regulation in the cell. As one example, multiple oncogenes or signaling pathways in a cancer cell could be targeted by multifunctional shRNAs.

There is, thus, a need in the art for improved methods of modulating gene expression, e.g. for use in the depletion or removal of unwanted cells from a patient, e.g. in cancer therapy. The present invention discloses means and methods to comply with the aforementioned needs.

Accordingly, the present invention relates to a polynucleotide forming a stem-loop structure under physiological conditions, wherein the loop comprises at least 80% of a sequence complementary or essentially complementary to a sequence of at least 15 contiguous nucleotides of an RNA expressed from a gene of interest.

The term "stem-loop structure" is known to the skilled artisan and relates to a structure of a polynucleotide comprising a double-stranded region, preferably including the 5' end and the 3' end of the polynucleotide and/or regions of the polynucleotide close to said ends (the stem), as well as at least one single-stranded, i.e. non-base-paired region (the loop). It is understood that the loop may comprise base-paired, i.e. double-stranded, regions, as well.

The term "physiological conditions" relates to internal or external conditions, preferably including temperature, pH, and solute concentration, corresponding to or mimicking conditions in a cell or in an organism. Thus, preferably, the physiological conditions are conditions in a solution, i.e. in a physiological solution. Preferably, the temperature is 10 - 50°C, more preferably 20 - 40°C, most preferably 25-38 °C. Preferably, the pH is 4 - 10, more preferably 5-9, most preferably 6 - 8. Preferably, the solute concentration is 0.1% - 5% (w/v), more preferably 0.25% - 4% (w/v), most preferably 0.5% - 3% (w/v). It is understood by the skilled person that the appropriate solute concentration of a physiological solution will depend on the kind of solute or solutes used. From the above, it is clear that a wide range of conditions or solutions may be used as a physiological condition or a physiological solution, including, e.g., 0.9% saline, Hank's balanced salt solution, cell culture media like RPMI 1640 or DMEM, or the cytoplasm of a cell, preferably even pathological conditions within a cell, more preferably, within a tumor cell or within an organism.. More preferably, physiological conditions are 37°C, pH 7.4, and the following solute concentrations: 40 mM KCI, 4 mM Mg₂Cl, 5 mM DTT, 1 mM ATP, 0.2 mM GTP.

The term "gene of interest" is understood by the skilled person. A gene of interest may be any gene of which at least a part is transcribed into RNA. More preferably, the gene of interest is a gene expressed by at least one host cell as a protein. Preferably, the gene of interest is an eukaryotic, archeal or a bacterial gene. More preferably, the gene of interest is a gene of a eukaryotic cell, more preferably a mammalian cell, most preferably a human cell.

In a preferred embodiment, the gene of interest is a disease-related gene. The term disease-related gene, preferably, relates to a gene associated with disease. Preferably, a gene is associated with disease if a modulation of the expression of said gene has been correlated with disease. Preferably, said modulation of expression is an increase in expression or a hyperactivation of said gene of interest. A preferred disease is an immune-related disease, i.e. a disease involving aberrant activity of immune cells, e.g. an autoimmune disease like Pemphigus vulgaris, Vitiligo, Diabetes mellitus type 1, Hashimoto's thyroiditis, Addison's disease, Idiopathic thrombocytopenic purpura, lupus erythematosus, Sjögren's syndrome, scleroderma, rheumatoid arthritis, Coeliac disease, Crohn's Disease, or Myasthenia gravis, or allergic diseases.

More preferably, the disease is cancer, i.e. the disease-related gene is a cancer-related gene. Preferably, the cancer is selected from the list consisting of acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor.

The term "complementary" relates to the fact that two polynucleotides or two ends of one polynucleotide comprise nucleic acid sequences permitting base-pairing according to Watson-Crick base-pairing rules over a stretch of at least 15 contiguous nucleotides. The term "essentially complementary" relates to a complementarity of at least 80%, at least 85%, at least 90%, at least 95%, at least 98%, or at least 99%.. The percent complementarity values are, preferably, calculated by setting the number of base-paired nucleotides within a given stretch of a polynucleotide into relation to the total number of nucleotides within said stretch. It is understood from this description that one or both of said polynucleotides may comprise one or more nucleotides inserted into the sequence lacking a direct counterpart in the matching sequence, i.e. the base-pairing equivalent of an insertion mutation.

Alternatively, the percent complementarity values are calculated as follows: one of the sequences is inverse-complemented, i.e. the sequence of the polynucleotide is inversed and at the same time each nucleotide is replaced by its base-pairing counterpart according to the Watson-Crick-rules, and then the percent identity values are calculated, preferably over the entire sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit [Needleman and Wunsch (J. Mol. Biol. 48; 443-453 (1970)) and Smith and Waterman (Adv. Appl. Math. 2; 482-489 (1981))], which are part of the GCG software packet [Genetics Computer Group, 575 Science Drive, Madison, Wisconsin, USA 53711 (1991)], are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

The polynucleotide of the present invention shall be provided, preferably, either as an isolated polynucleotide or in genetically or chemically modified form. The polynucleotide, preferably, is an RNA polynucleotide, more preferably an shRNA or a precursor of a miRNA. The term encompasses single- or double-stranded, more preferably partially double-stranded polynucleotides. Moreover, comprised are also chemically modified polynucleotides including naturally occurring modified polynucleotides, such as glycosylated or methylated polynucleotides or artificially modified ones such as biotinylated polynucleotides. Preferably, the polynucleotide is an artificial polynucleotide, i.e. a polynucleotide whose sequence has not been described, more preferably a polynucleotide whose sequence is not occurring in nature.

The loop comprised in the polynucleotide of the present invention comprises at least a part of a sequence complementary or essentially complementary (a "targeting sequence") to a gene of interest. Preferably, the loop comprises at least 80%, more preferably at least 90%, most preferably at least 95% of said targeting sequence. It is understood that the residual part of said targeting sequence is, preferably, comprised in the stem. Preferably, the targeting sequence is complementary or essentially complementary to a sequence of at least 10, 12, 15, 17, 18, 19, or 20 contiguous nucleotides of an RNA expressed from a gene of interest (a "target sequence"). More preferably, the targeting sequence is complementary or essentially complementary to a sequence of 17-25, still more preferably 19-23, and most preferably 20-22 contiguous nucleotides of a target sequence.

In a preferred embodiment, the loop of the polynucleotide of the present invention comprises 5 to 50, 9-40, 12-35, or 15-30, more preferably 17-28 nucleotides. Preferably, at least 50%, at least 60%, at least 70%, at least 80%, at least 90%, at least 95%, at least 98% of said nucleotides comprised in the loop are non-base-paired, more preferably under physiological conditions as described herein above.

In a preferred embodiment, the stem of the polynucleotide of the present invention comprises at least one additional targeting sequence, i.e. a sequence complementary or essentially complementary to a sequence comprised in or constituting an RNA expressed from a gene of interest. It is understood that the targeting sequence may be identical, i.e. the same, or a different sequence as compared to the targeting sequence comprised in the loop of the polynucleotide. Preferably, the target sequences are comprised in the same gene of interest, more preferably, the target sequences are comprised in different genes of interest, e.g. in different genes of interest related to the same disease. In a further preferred embodiment, the stem and/or the loop of the polynucleotide comprise more than one target sequence. It is understood by the skilled person that the polynucleotide may be multi-functional in that it may allow for modulation of expression of more than one gene of interest.

In an embodiment, the polynucleotide of the present invention is for use in the treatment of disease as defined herein, i.e. is for use as a medicament. In a further embodiment, the polynucleotide of the present invention is for use in the treatment of cancer and/or immune-related disease. Thus, the present invention relates to the use of the polynucleotide of the present invention for the manufacture of a medicament. Also, the present invention relates to the use of the polynucleotide of the present invention for the manufacture of a medicament for treating cancer and/or immune-related disease.

Advantageously, it was found in the work underlying the present invention that not only sequences comprised in the stem of an RNA stem-loop are processed to become incorporated into the cellular RISC complex, but also sequences comprised in the loop are processed apparently along the same or a similar pathway and, thus, can lead to gene silencing.

The definitions made above apply mutatis mutandis to the following:

The present invention further relates to a vector comprising or encoding a polynucleotide according to the present invention.

The term "vector", preferably, encompasses phage, plasmid, viral or retroviral vectors as well artificial chromosomes, such as bacterial or yeast artificial chromosomes. Moreover, the term also relates to targeting constructs which allow for random or site- directed integration of the targeting construct into genomic DNA. Such target constructs, preferably, comprise DNA of sufficient length for either homologous or heterologous recombination as described in detail below. The vector encompassing or encoding the polynucleotide of the present invention, preferably, further comprises selectable markers for propagation and/or selection in a host. The vector may be incorporated into a host cell by various techniques well known in the art. For example, a plasmid vector can be introduced in a precipitate such as a calcium phosphate precipitate or rubidium chloride precipitate, or in a complex with a charged lipid or in carbon-based clusters, such as fullerenes. Alternatively, a plasmid vector may be introduced by heat shock or electroporation techniques. Should the vector be a virus, it may be packaged in vitro using an appropriate packaging cell line prior to application to host cells. Retroviral vectors may be replication competent or replication defective. In the latter case, viral propagation generally will occur only in complementing host/cells.

More preferably, in the vector of the invention the polynucleotide is encoded on a DNA operatively linked to expression control sequences allowing expression in prokaryotic or eukaryotic cells or isolated fractions thereof. Expression of said polynucleotide comprises transcription of the polynucleotide, producing RNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional enhancers. Possible regulatory elements permitting expression in prokaryotic host cells comprise, e.g., the lac, trp or tac promoter in E. coli, and examples for regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Moreover, inducible expression control sequences may be used in an expression vector encompassed by the present invention. Such inducible vectors may comprise tet or lac operator sequences or sequences inducible by heat shock or other environmental factors. Suitable expression control sequences are well known in the art. Beside elements which are responsible for the initiation of transcription such regulatory elements may also comprise transcription termination signals, such as the SV40-poly-A site or the tk-poly-A site, downstream of the polynucleotide. In this context, suitable expression vectors are known in the art such as Okayama-Berg cDNA expression vector pcDV1 (Pharmacia), pBluescript (Stratagene), pCDM8, pRc/CMV, pcDNA1, pcDNA3 (Invitrogen) or pSPORT1 (GIBCO BRL). Preferably, said vector is an expression vector and a gene transfer or targeting vector. Expression vectors derived from viruses such as retroviruses, vaccinia virus, adeno-associated virus, herpes viruses, or bovine papilloma virus, may be used for delivery of the polynucleotide or vector of the invention into a targeted cell population. Methods which are well known to those skilled in the art can be used to construct recombinant viral vectors; see, for example, the techniques described in Sambrook, Molecular Cloning A Laboratory Manual, Cold Spring Harbor Laboratory (1989) N.Y. and Ausubel, Current Protocols in Molecular Biology, Green Publishing Associates and Wiley Interscience, N.Y. (1994).

The present invention also relates to a host cell comprising the polynucleotide and/or the vector according to the present invention.

The term "host cell", as used herein, relates to a cell suitable for propagation of a vector of the present invention and/or for expressing and/or taking up and processing the polynucleotide of the present invention. Preferably, the host cell is a bacterial or an archeal cell. More preferably, the host cell is an eukaryotic cell, still more preferably a mammalian cell, e.g. a mouse, rat, dog, cat, sheep, cow, goat, guinea pig, monkey, or a swine cell, most preferably a human cell.

In an embodiment, the vector and/or the host cell of the present invention are for use in the treatment of disease as defined herein, i.e. is for use as a medicament. In further embodiments, the vector and/or the host cell of the present invention are for use in the treatment of cancer and/or immune-related disease. Thus, the present invention relates to the use of the vector and/or the host cell of the present invention for the manufacture of a medicament. Also, the present invention relates to the use of the vector and/or the host cell of the present invention for the manufacture of a medicament for treating cancer and/or immune-related disease.

The present invention also relates to the use of a polynucleotide, a vector, and/or a host cell according to the present invention for treating disease.

Further, the present invention relates to a method of inhibiting an unwanted cell population, comprising (i) contacting said cell population with a polynucleotide, a vector, or a host cell according to the present invention, and (ii) thereby inhibiting an unwanted cell population.

The method of the present invention, preferably, is an in vitro method. Moreover, it may comprise steps in addition to those explicitly mentioned above. For example, further steps may relate, e.g., to identifying, obtaining, and/or pretreating a cell population for step i), or adding additional treatment steps before or in step ii). Moreover, one or more of said steps may be performed by automated equipment.

As used herein, the term "unwanted cell population" relates to a population of cells, preferably host cells, of which it is desirable that their proliferation is inhibited, i.e. stopped or slowed down, or that the cells are destroyed. Preferably, an unwanted cell population is a cell population causing an adverse effect, preferably disease, in a subject.

As used herein, the term "subject", preferably, relates to a vertebrate, more preferably a mammal, most preferably a human.

The term "contacting" as used in the context of the methods of the present invention is understood by the skilled person. Preferably, the term relates to bringing a compound (polynucleotide, vector, or host cell) of the present invention in physical contact with a cell and thereby, e.g. allowing the compound and the cell to interact.

The present invention also relates to a kit comprising a polynucleotide, a vector, and/or a host cell according to the present invention, and an instruction manual.

The term "kit" as used herein refers to a collection of the aforementioned means, e.g., a composition comprising a polynucleotide of the present invention and/or means for contacting a subject, a host cell, or an unwanted cell population, preferably, provided separately or within a single container. The container, also preferably, comprises instructions for carrying out the method of the present invention. The components of the kit are provided, preferably, in a "ready-to-use" manner, e.g., concentrations are adjusted accordingly, etc.

The present invention further relates to a device comprising a polynucleotide, a vector, and/or a host cell according to the present invention and, preferably, means for applying said polynucleotide, vector, and/or host cell to the body of a subject.

The term "device", as used herein, relates to a system of means comprising at least the means operatively linked to each other as to allow administration of the compound or of the medicament of the present invention. Preferred means for administering medicaments are well known in the art. How to link the means in an operating manner will depend on the type of means included into the device and on the kind of administration envisaged. Preferably, the means are comprised by a single device in such a case. Said device may accordingly include a delivery unit for the administration of the compound or medicament and a storage unit for storing said compound or medicament until administration. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician. In a preferred embodiment, the device is a syringe, more preferably with a needle, comprising the compound or medicament of the invention. In another preferred embodiment, the device is an intravenous infusion (IV) equipment comprising the compound or medicament. In another preferred embodiment, the device is an endoscopic device comprising the compound or medicament for flushing a site of tumor resection before and/or after surgical resection of a tumor. In still another preferred embodiment the device is an inhaler comprising the compound of the present invention, wherein, more preferably, said compound is formulated for administration as an aerosol.

Also, the present invention relates to a method of treating or preventing disease in a subject afflicted with said disease, comprising (i) contacting said subject with a polynucleotide, a vector, or a host cell according the present invention, and (ii) thereby treating said disease in a subject afflicted with said disease.

All references cited in this specification are herewith incorporated by reference with respect to their entire disclosure content and the disclosure content specifically mentioned in this specification.

### Figure Legends

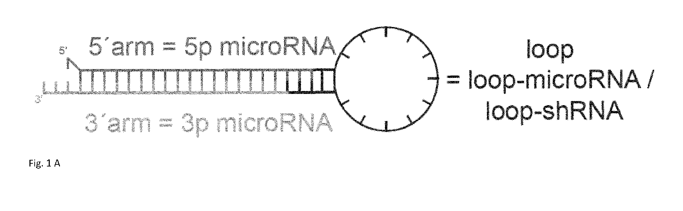

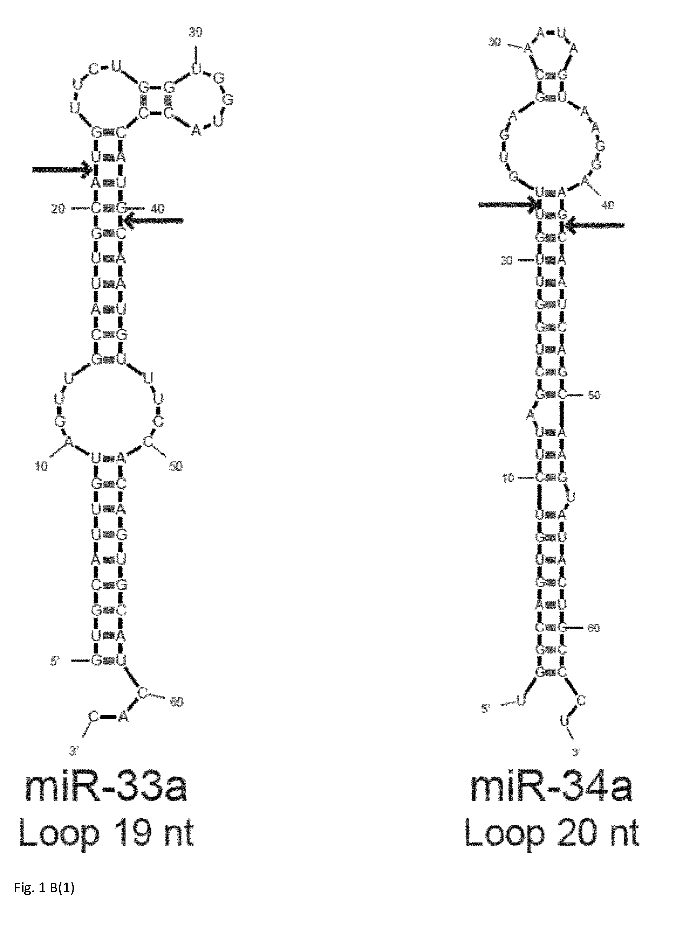

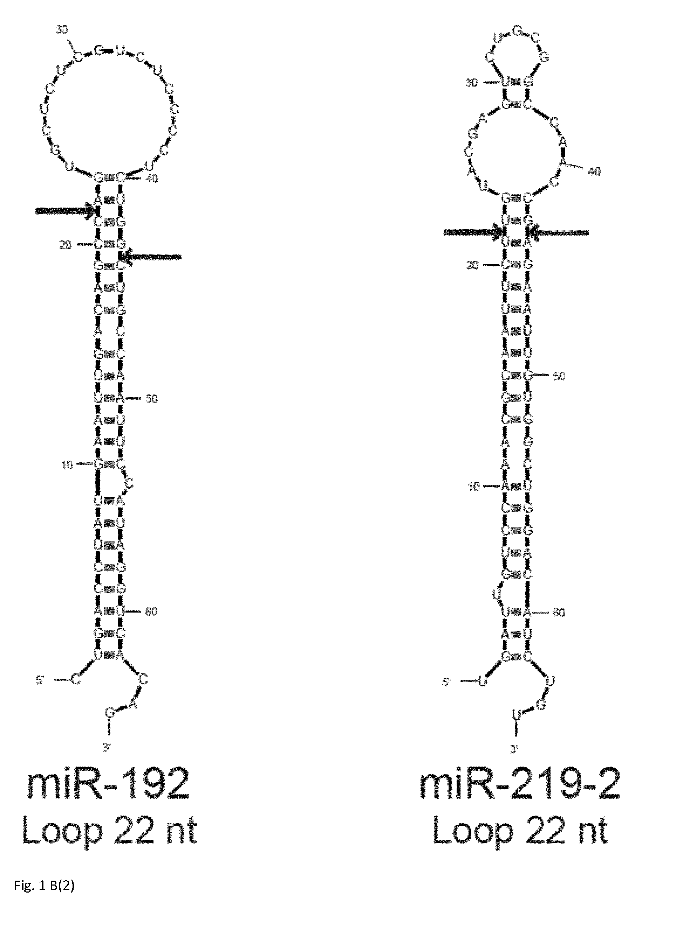

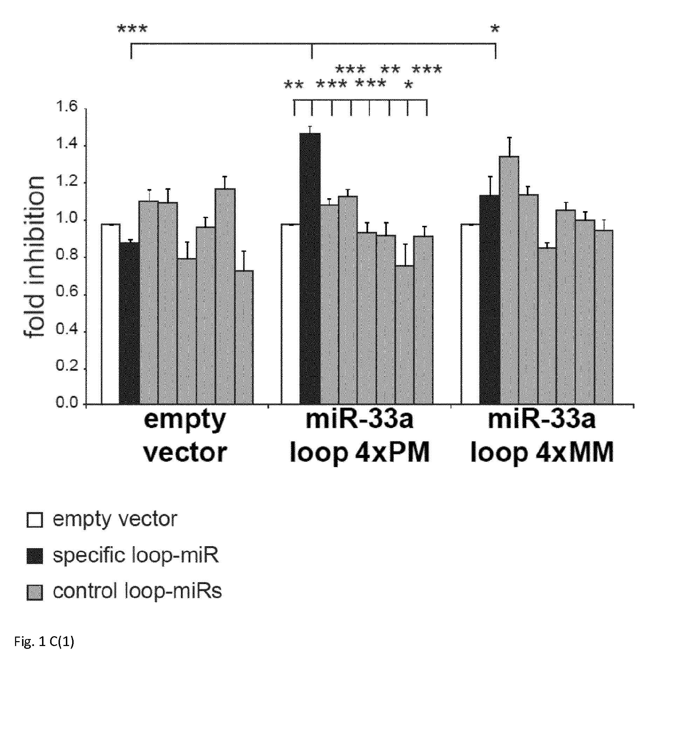

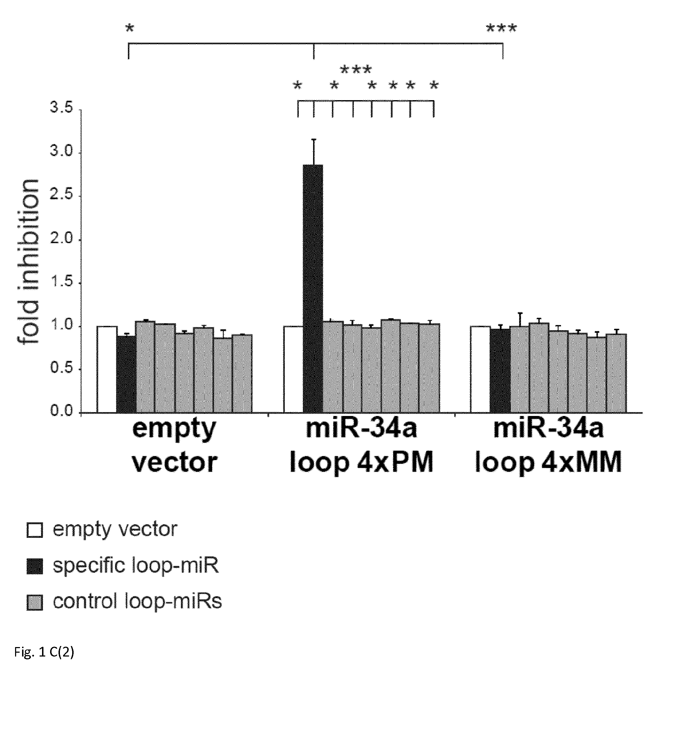

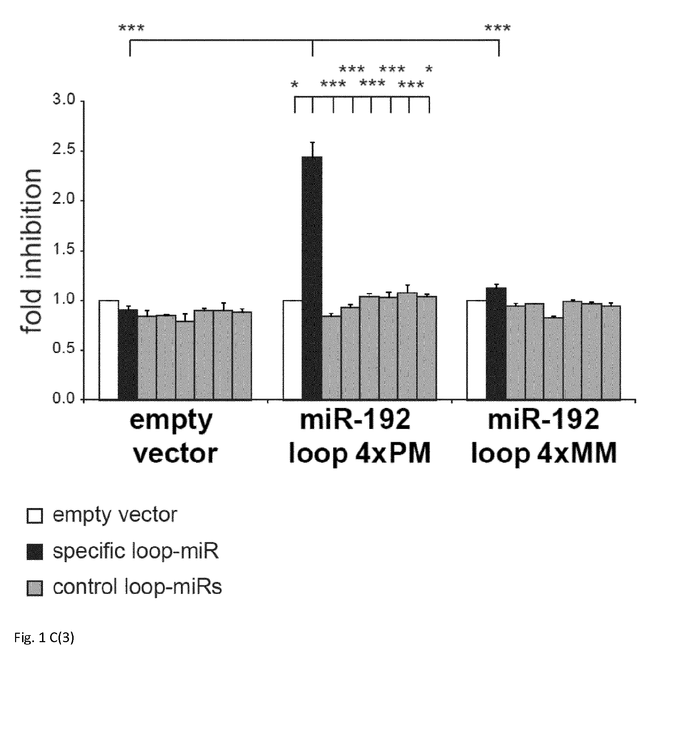

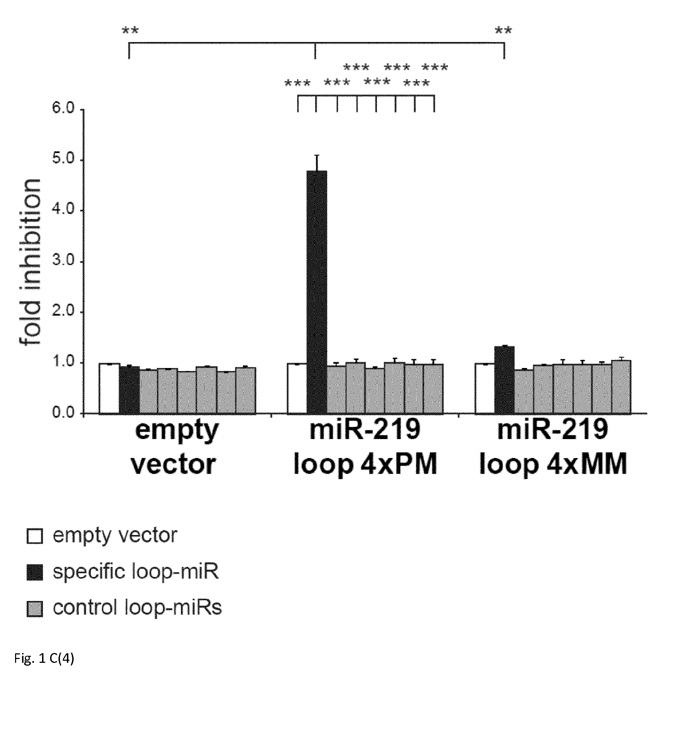

Fig. 1: Discovery and activity of endogenous Loop-miRNAs
   (A) Stem-loop RNA hairpins - endogenous as well as synthetic sequences - can be processed by RNAses into three molecules: The 5p- and 3p-arm of the hairpin stem can give rise to the well-known active microRNAs or siRNAs. However, we discovered endogenous and invented synthetic small RNA sequences that are not derived form the hairpin stem, but from the hairpin loop region. Thus, multiple independent small and active RNAs can be generated from a short hairpin RNA: three single-stranded short RNA molecules: the 5' arm (5p), the 3' arm (3p) and the loop. (B) Selected examples of endogenous loop-miR-generating pre-miRNA structures were predicted using Mfold, verifying single-stranded loop regions of different lengths. (C) The activity of natural loop-derived small RNAs is shown: HEK293 cells were cotransfected with the plasmid derived loop precursors hairpin of different microRNAs and a reporter construct encoding luciferase fused to four perfectly matched binding sites (4x PM) for the loop region of miR-33a, miR-34a, miR-192 and miR-219-2. To verify the specificity of the assay, control miRNAs with divergent seed regions were transfected, as well (grey bars). Furthermore, constructs with mismatches in the seed region of the binding sites (4x MM) or the empty vectro with luciferase only without any binding sites served as negative control. Depicted is the mean fold inhibition (+SEM) of the respective targets compared to control/empty vector transfection.

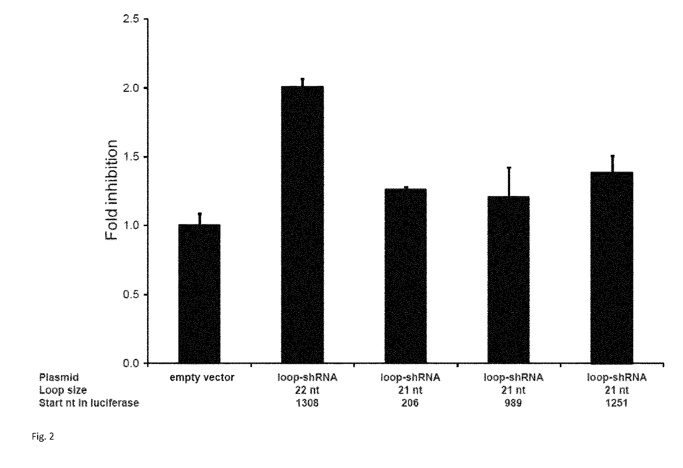

Fig. 2: Invention and development of an artificial Loop-shRNA
   Four artificial loop-shRNAs were invented and developed that target the luciferase gene as a proof-of-principle experiment. All Loop-shRNAs showed inhibitory activity towards the firefly luciferase expression as determined in luciferase assays. These four sequences were selected independently and target different regions of the firefly luciferase gene.

The following Examples shall merely illustrate the invention. They shall not be construed, whatsoever, to limit the scope of the invention.

### Loop-shRNA Invention & Development

The sequences of the hsa-miRNAs are publicly available in the miRBase database (Griffiths-Jones S., NAR 2004 32(Database Issue):D109-D111; Kozomara A, Griffiths-Jones S., NAR 2011 39(Database Issue):D152-D157) and have been included in the sequence listing under the following reference numbers: miR-33a: SEQ ID NO:1, miRBase ACC NO: MI0000091, loop-RNA comprised therein: SEQ ID NO:2; miR-34a: SEQ ID NO:3, miRBase ACC NO: MI0000268, loop-RNA comprised therein: SEQ ID NO:4; miR-192: SEQ ID NO:5, miRBase ACC NO: MI0000268, loop-RNA comprised therein: SEQ ID NO:6; and miR-219-2: SEQ ID NO:7, miRBase ACC NO: MI0000740, loop-RNA comprised therein: SEQ ID NO:8. The sequences in the database show the complete stem-loop sequences, whereas the sequences in the sequence listing (SEQ ID NOs: 1, 3, 5, 7) only show the sequence starting with the 5' nucleotide of the 5p fragment down to the 3' nucleotide of the 3p fragment.

We developed artificial loop structures that were 21 or 22 nucleotides long and were fully complementary to the firefly luciferase gene. The transfection of these loop-shRNAs - in the form of a plasmid fused to the miR-219-2 hairpin stem - decreased the expression of the luciferase proving its activity. The silencing activity was between 21% and 101% targeting different parts of the luciferase gene starting at nucleotides 206, 989, 1251 or 1308. The sequences of the constructs used for the experiments shown in Fig. 2 have been included in the sequence listing. loop-shRNA/21nt/206: SEQ ID NO:9, loop-RNA comprised therein: SEQ ID NO:10; loop-shRNA/21nt/989: SEQ ID NO:11, loop-RNA comprised therein: SEQ ID NO:12; loop-shRNA/21nt/1251: SEQ ID NO13, loop-RNA comprised therein: SEQ ID NO:14; loop-shRNA/22nt/1308: SEQ ID NO:15, loop-RNA comprised therein: SEQ ID NO:16.

### Cell lines and transfection

HEK293 cells were cultured in DMEM + 10% FCS + Glutamine (Sigma, Taufkirchen, Germany) at 37°C in 5% CO₂. For plasmid transfection, cells were seeded to reach 90% confluence at the day of transfection and 3 µg plasmid DNA were transfected with 10 µl Polyethylenimine (PEI) in TBS buffer per well in 6-well plates.

### Expression plasmid cloning

Expression plasmids for endgenous loop-miRs (Fig. 1) and for the synthetic loop-shRNAs were cloned into the pcDNA3.1D-TOPO vector (Invitrogen) by TOPO cloning.

Luciferase constructs were generated using the psiCheck2 vector (Promega, Mannheim, Germany) and contained four perfectly matched (4 x PM) or four mismatched control (4x MM) binding sites to sense the activity of the newly discovered andogenous and the newly invented synthetic loop-RNAs.

### Luciferase assays

HEK293 cells were seeded to reach 80% confluence at the day of transfection and transfected with 1.2 µg plasmid DNA and 4.0 µl PEI (1 mg/ml) in a 24-well plate. Each transfection contained 200 ng psiCheck2-Firefly expression construct including the 3'-UTR of interest, 500 ng expression plasmid for the miRNA of interest and 500 ng Ago2 expression plasmid. After 72 hours, cells were lysed in 150 µl Passive Lysis Buffer (Promega, Mannheim). 20 µl lysate were subsequently analyzed using 50 µl LAR II substrate or Stop&Glow (1:3 dilutions) to determine Firefly and Renilla luciferase activity, respectively (Dual Luciferase Reporter Assay System; Promega). Each sample was analyzed in quadruplicates, and each transfection was carried out at least three times. Firefly activity was normalized to Renilla activity, and mean values plus standard error of mean are depicted. Reporter constructs encoding luciferase fused to a 3'-UTR with mismatches in the seed region served as negative controls.

## Claims

1. A polynucleotide forming a stem-loop structure under physiological conditions, wherein the loop comprises at least 80% of a sequence complementary or essentially complementary to a sequence of at least 15 contiguous nucleotides of an RNA expressed from a gene of interest.

2. The polynucleotide of claim 1, wherein the polynucleotide is an artificial polynucleotide.

3. The polynucleotide of claim 1 or 2, wherein the loop consists of 5 to 50 nucleotides of which at least 70% are non-basepaired nucleotides.

4. The polynucleotide of any one of claims 1 to 3, wherein the loop comprises at least 80% of a sequence complementary or essentially complementary to a sequence of at least 17 contiguous nucleotides of an RNA expressed from a gene of interest.

5. The polynucleotide of any one of claims 1 to 4, wherein the gene of interest is a disease-related gene.

6. The polynucleotide of claim 5, wherein the disease-related gene is a cancer-related gene.

7. The polynucleotide of any one of claims 1 to 7, wherein the stem comprises the same or a different sequence complementary or essentially complementary to a sequence of at least 10 contiguous nucleotides of an RNA expressed from the same or a different gene of interest.

8. A vector comprising or encoding a polynucleotide according to any one of claims1 to 7.

9. A host cell comprising the polynucleotide of any one of claims 1 to 7 and/or the vector of claim 8.

10. A polynucleotide according to any one of claims 1 to 7, a vector according to claim 8, or a host cell according to claim 9, for use in the treatment of disease.

11. A polynucleotide according to any one of claims 1 to 7, a vector according to claim 8, or a host cell according to claim 9, for use in the treatment of cancer and/or immune-related disease.

12. Use of a polynucleotide according to any one of claims 1 to 7, a vector according to claim 8, or a host cell according to claim 9, for treating disease.

13. A method of inhibiting an unwanted cell population, comprising
(i) contacting said cell population with a polynucleotide according to any one of claims 1 to 7, a vector according to claim 8, or a host cell according to claim 9, and
(ii) thereby inhibiting an unwanted cell population.

14. A kit comprising a polynucleotide according to any one of claims 1 to 7, a vector according to claim 8, and/or a host cell according to claim 9, and an instruction manual.

15. A device comprising a polynucleotide according to any one of claims 1 to 7, a vector according to claim 8, and/or a host cell according to claim 9 and, preferably, means for applying said polynucleotide, vector, and/or host cell to the body of a subject.
